# EUROPEAN PATENT APPLICATION

(11) **EP 1 595 889 A1**
(43) Date of publication of application: **16.11.2005**
(21) Application number: 03815854.9
(22) Date of filing: 03.04.2003
(51) Int. Cl.: C07K 1/113, C07K 7/06, C07K 7/08, G01N 33/68, G01N 27/62

(54) **METHOD OF ELIMINATING PHOSPHATE GROUP OF PEPTIDE AND METHOD OF ANALYZING PEPTIDE**

(30) Priority: 14.02.2003 JP 2003036472
(71) Applicant: Shimadzu Corporation, Kyoto-shi, Kyoto 604-8511 (JP)
(72) Inventor: KUYAMA, Hiroki, c/o Shimadzu Corporation, Kyoto-shi, Kyoto 604-8511 (JP); TODA, Chikako, c/o Shimadzu Corporation, Kyoto-shi, Kyoto 604-8511 (JP); NISHIMURA, Osamu, c/o Shimadzu Corporation, Kyoto-shi, Kyoto 604-8511 (JP)
(74) Representative: Pohlmann, Eckart
(86) International application number: PCT/JP2003/004305
(87) International publication number: WO 2004/072106

(57) **Abstract**

The present invention provides a method for eliminating phosphate groups of peptides that relies not on enzymes but on a chemical treatment, and an effective method for peptide analysis that uses such a method. A method for eliminating a phosphate group of a peptide, the method comprising the use of a reagent containing at least one selected from the group consisting of hydrogen fluoride, hydrofluoric acid, and a hydrogen fluoride-containing compound, and a method for peptide analysis that uses such a method. As the hydrogen fluoride-containing compound, hydrogen fluoride-pyridine is preferably used. The elimination of the phosphate group from a peptide may be carried out so that the total amount of the hydrogen fluoride, hydrogen fluoride in the hydrofluoric acid, and hydrogen fluoride in the hydrogen fluoride-containing compound contain in the reagent is 10 to 100wt% with respect to the reagent, and the temperature for the elimination reaction is -10 to 50°C. Further, the peptide analysis may be performed by mass spectrometry with the use of MALDI-TOFMS.

## Description

### TECHNICAL FIELD

The present invention relates to protein chemistry and mass spectrometry of peptides/proteins.

### BACKGROUND ART

After transcribed and translated from the genome, most proteins undergo modification (i.e., post-translational modification). This modification controls the proteins' activities and functions. Various post-translational modification reactions include phosphorylation, sulfation, acetylation, glycosylation, and modification with lipids. Of these, phosphorylation reactions play a particularly important role in the control of intracellular signal transduction, intracellular metabolic activity, cell cycle, and the like. Therefore, analyses of proteins that have post-translational modification form have great importance in terms of understanding protein functions.

Analysis of phosphorylated proteins often involves dephosphorylation. Traditionally, the reaction has been carried out by using an enzyme, such as alkaline phosphatase. In some proteins, however, the substrate specificity, structural dependence of the enzyme and the like result in incomplete dephosphorylation.

In comparison, hydrofluoric acid has been used in the dephosphorylation of phosphorylated sugar chains (See, for example, non-patent articles 1 through 3).

Non-patent article 1: E. Fuchs and C. Gilvarg, Analytical Biochemistry (US), 90(1978), pp. 465-473

Non-patent article 2: C. J. Lee and B. A. Fraser, Journal of Biological Chemistry (US), 255(1980), pp. 6847-6853

Non-patent article 3: L. R. Phillips, O. Nishimura, and B. A. Fraser, Carbohydrate Research (US), 121(1983), pp. 243-255

### DISCLOSURE OF THE INVENTION

### Object of the Invention

Accordingly, it is an objective of the present invention to provide a method for eliminating phosphate groups of peptides that relies not on enzymes but on a chemical treatment. It is also an objective of the present invention to provide an effective method for peptide analysis that uses such a method.

### Summary of the Invention

In the course of studies, the present inventors have found that the aforementioned objectives can be achieved by reacting a peptide with a reagent that contains at least one selected form the group consisting of hydrogen fluoride, hydrofluoric acid, and a hydrogen fluoride-containing compound. This finding ultimately led the present inventors to complete the present invention.

Accordingly, the present invention is a method of eliminating a phosphate group of a peptide, the method comprising the use of a reagent containing at least one selected from the group consisting of hydrogen fluoride, hydrofluoric acid, and a hydrogen fluoride-containing compound.

As used herein, the term "peptide" is intended to encompass proteins.

The present invention is the above-described method of eliminating a phosphate group of a peptide, wherein the hydrogen fluoride-containing compound is hydrogen fluoride-pyridine.

The present invention is the above-described method of eliminating a phosphate group of a peptide, wherein the total amount of the hydrogen fluoride, hydrogen fluoride in the hydrofluoric acid, and hydrogen fluoride in the hydrogen fluoride-containing compound contain in the reagent is 10 to 100wt% with respect to the reagent.

The present invention is the above-described method of eliminating a phosphate group of a peptide, wherein the temperature for the elimination reaction is -10 to 50°C.

The present invention is the above-described method of eliminating a phosphate group of a peptide, wherein the elimination reaction is carried out as a liquid phase reaction or a gas phase reaction.

The present invention is a method of analyzing a peptide that uses the above-described method of eliminating a phosphate group of a peptide.

The present invention is the above-described method of analyzing a peptide comprising the use of mass spectrometry.

As the mass spectrometry, matrix-assisted laser desorption ionization (MALDI) and time of flight mass spectrometry (TOFMS) are preferably used.

The present invention is a novel compound comprising a peptide identified by eliminating a phosphate group of a peptide using a reagent containing at least one selected from the group consisting of hydrogen fluoride, hydrofluoric acid, and a hydrogen fluoride-containing compound.

The present invention is a candidate compound for a pharmaceutical product developed from the above-described novel compound.

### BRIEF DESCRIPTION OF THE DRAWINGS

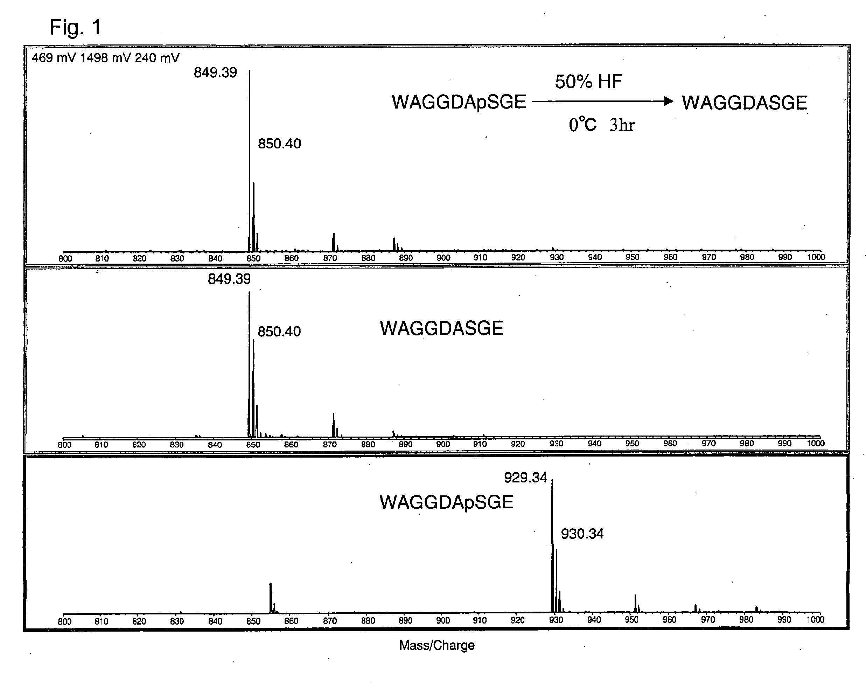

Fig. 1 is a comparison of MALDI-TOF mass spectra of a phosphopeptide of SEQ ID NO: 1 of the sequence listing; a peptide of SEQ ID NO: 2 of the sequence listing, which was obtained by dephosphorylation with hydrogen fluoride of the phosphopeptide of SEQ ID NO:1; and a separately prepared peptide having an identical sequence to the peptide of SEQ ID NO:2.

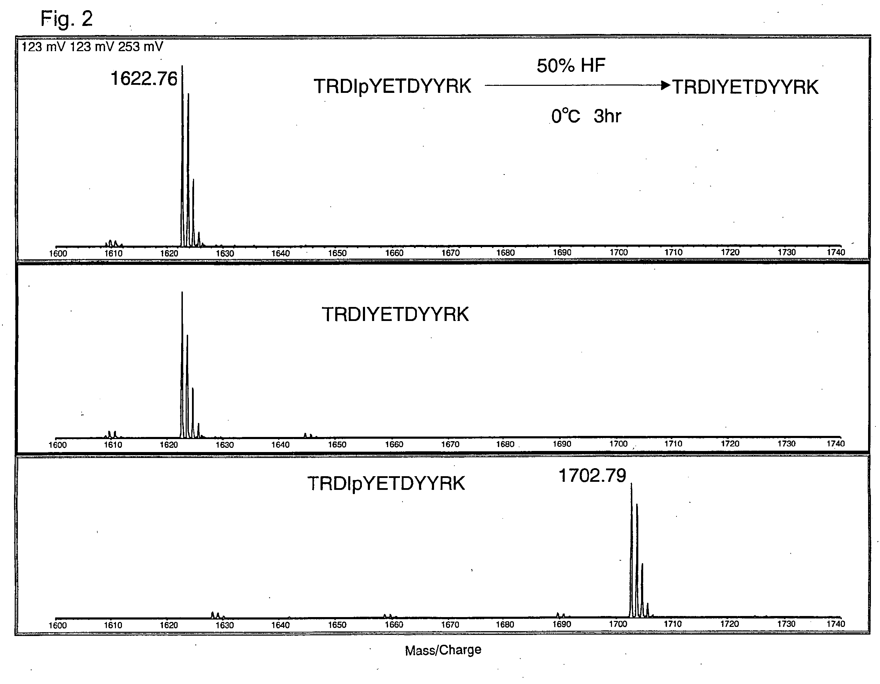

Fig. 2 is a comparison of MALDI-TOF mass spectra of a phosphopeptide of SEQ ID NO: 3 of the sequence listing; a peptide of SEQ ID NO: 4 of the sequence listing, which was obtained by dephosphorylation with hydrogen fluoride of the phosphopeptide of SEQ ID NO: 3; and a separately prepared peptide having an identical sequence to the peptide of SEQ ID NO: 4.

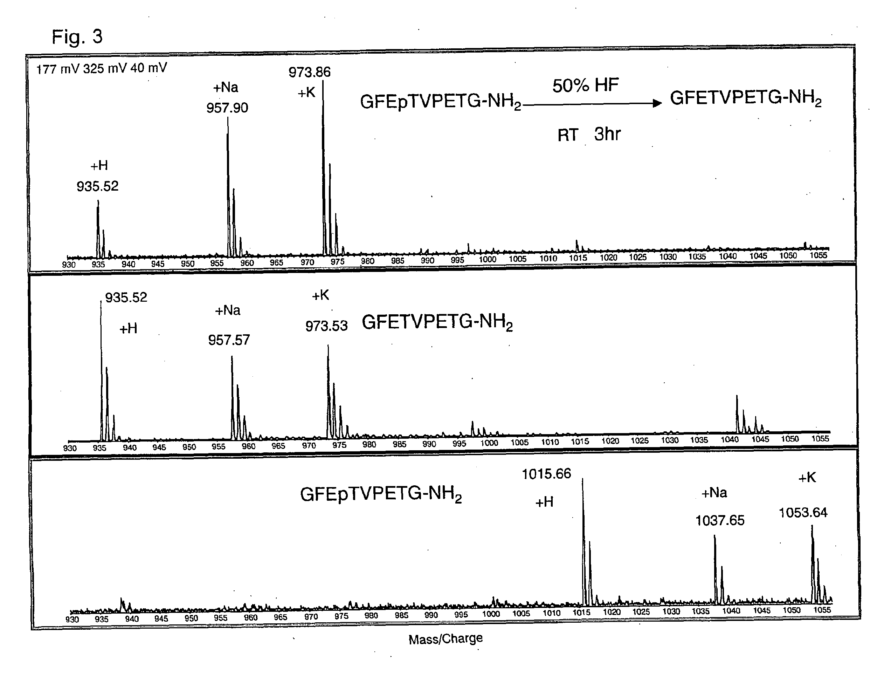

Fig. 3 is a comparison of MALDI-TOF mass spectra of a phosphopeptide of SEQ ID NO: 5 of the sequence listing; a peptide of SEQ ID NO: 6 of the sequence listing, which was obtained by dephosphorylation with hydrogen fluoride of the phosphopeptide of SEQ ID NO: 5; and a separately prepared peptide having an identical sequence to the peptide of SEQ ID NO: 6.

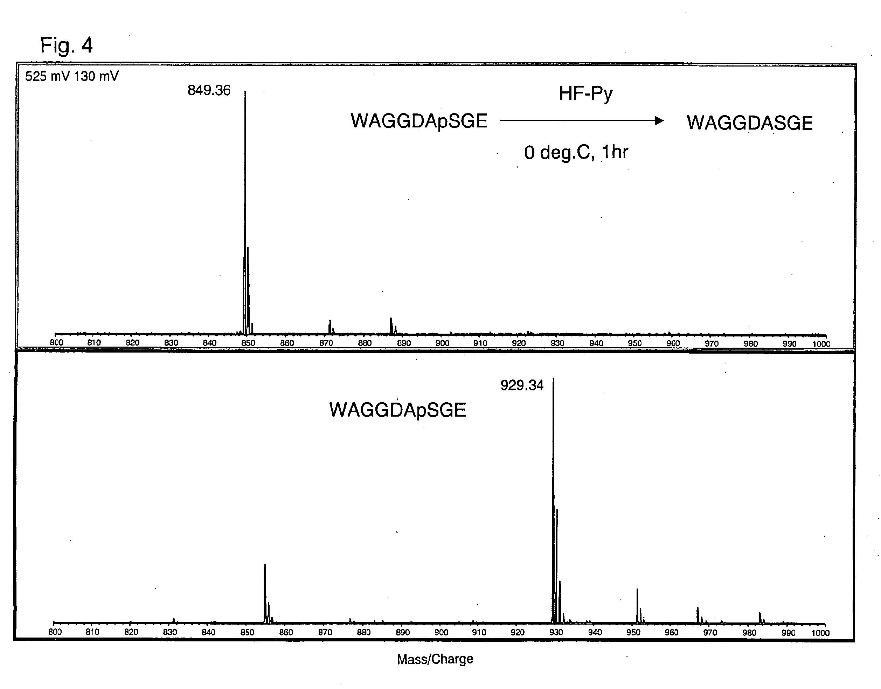

Fig. 4 is a comparison of MALDI-TOF mass spectra of a phosphopeptide of SEQ ID NO: 1 of the sequence listing; and a peptide of SEQ ID NO: 2 of the sequence listing, obtained by dephosphorylation with hydrogen fluoride-pyridine of the phosphopeptide of SEQ ID NO: 1.

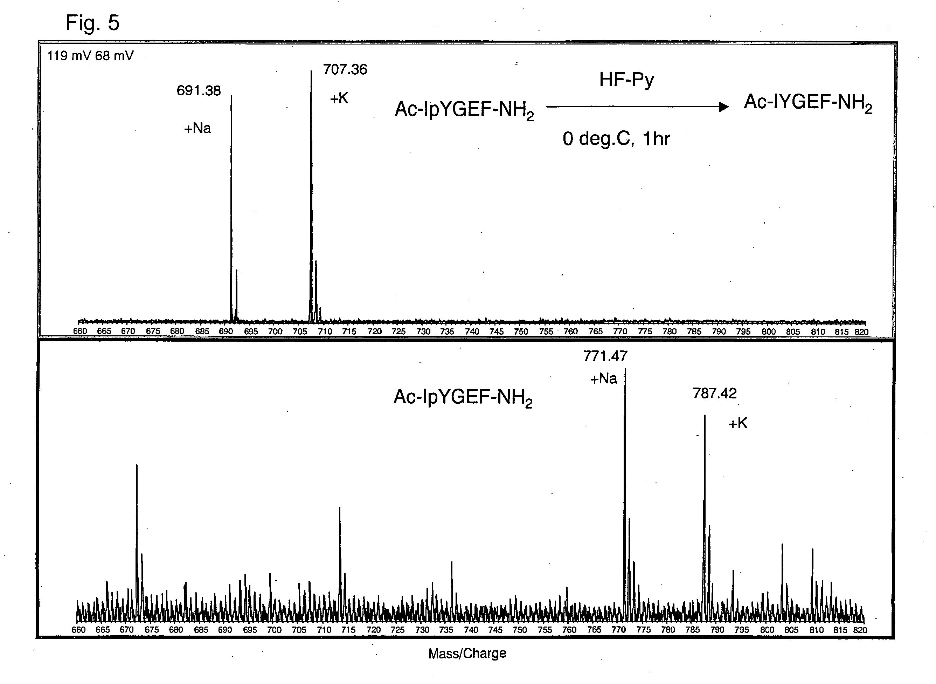

Fig. 5 is a comparison of MALDI-TOF mass spectra of a phosphopeptide of SEQ ID NO: 7 of the sequence listing; and a peptide of SEQ ID NO: 8 of the sequence listing, obtained by dephosphorylation with hydrogen fluoride-pyridine of the phosphopeptide of SEQ ID NO: 7.

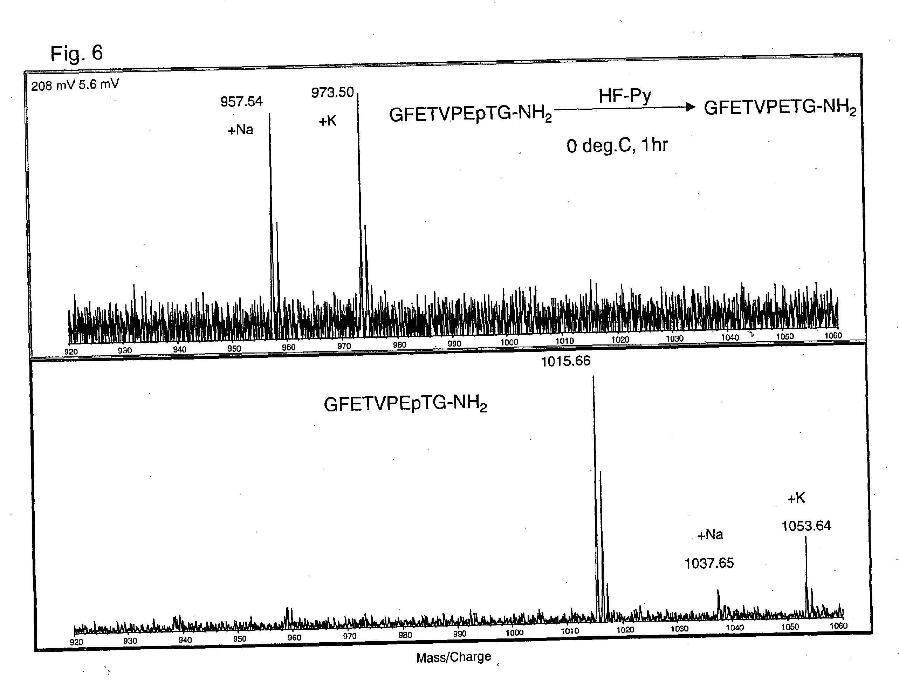

Fig. 6 is a comparison of MALDI-TOF mass spectra of a phosphopeptide of SEQ ID NO: 9 of the sequence listing; and a peptide of SEQ ID NO: 10 of the sequence listing, obtained by dephosphorylation with hydrogen fluoride-pyridine of the phosphopeptide of SEQ ID NO: 9.

### MODES FOR CARRYING OUT THE INVENTION

According to the present invention, a reagent that contains at least one selected from the group consisting of hydrogen fluoride, hydrofluoric acid, and a hydrogen-fluoride containing compound is used to eliminate the phosphate group of a peptide: this reagent serves as a dephosphorization agent in the present invention.

The type of phosphate is not limited to monophosphate. Namely, the type of phosphate includes monophosphate, diphosphate and, theoretically, triphosphate.

As opposed to the techniques using enzymes, which exhibit substrate specificity and structural dependence, the present invention is a chemical process and brings dephosphorylation to completion irrespective of the type of peptide. Thus, the present invention is applicable to any kind of peptide, offering a significant advantage.

In the method of the present invention, the above-described reagent is reacted with a peptide. The reagent contains at least one selected from the groups consisting of hydrogen fluoride, hydrofluoric acid, and a hydrogen fluoride-containing compound. One example of the hydrogen fluoride-containing compound is hydrogen fluoride-pyridine. This reagent may be used either in a solvent-free form or as a solution.

The reaction may be carried out as a liquid phase reaction or a gas phase reaction.

The reagent may be one or a combination of any two selected from the group consisting of hydrogen fluoride, hydrofluoric acid, and a hydrogen fluoride-containing compound.

When the reagent contains one selected from the group consisting of hydrogen fluoride, hydrofluoric acid, and a hydrogen fluoride-containing compound, the amount of the hydrogen fluoride, hydrogen fluoride present in the hydrofluoric acid, or hydrogen fluoride present in the hydrogen fluoride-containing compound is preferably from 10wt% to 100wt%, more preferably from 10 to 80wt%, and still more preferably from 10 to 70wt% with respect to the reagent.

Namely, when hydrogen fluoride is to be selected, the reagent may be used in the form of either solvent-free hydrogen fluoride (100wt%) or a solution containing hydrogen fluoride. The solution containing hydrogen fluoride preferably is used so that the amount of the hydrogen fluoride is 10wt% or more and less than 100wt%, more preferably from 10 to 80wt%, and still more preferably from 10 to 70wt% with respect to the reagent. Of solutions containing hydrogen fluoride, aqueous solutions are particularly referred to as "hydrofluoric acid" in this description. When hydrofluoric acid is to be selected, the reagent may be used in the form of either a hydrofluoric acid or a solution containing hydrofluoric acid. In such a case, the amount of hydrogen fluoride present in the hydrofluoric acid is preferably 10wt% or more and less than 100wt%, more preferably from 10 to 80wt%, and still more preferably from 10 to 70wt% with respect to the reagent. When the hydrogen fluoride-containing compound is to be selected, the reagent may be used in the form of either a solvent-free hydrogen fluoride-containing compound or a solution containing hydrogen fluoride-containing compound. The solvent-free compound preferably contains hydrogen fluoride in an amount of 10wt% or more and less than 100wt%, more preferably from 10 to 80wt%, and still more preferably from 10 to 70wt% with respect to the reagent. The solution containing the hydrogen fluoride-containing compound preferably is used so that the amount of the hydrogen fluoride is 10wt% or more and less than 100wt%, more preferably from 10 to 80wt%, and still more preferably from 10 to 70wt% with respect to the reagent.

When the reagent contains a combination of any two selected from the groups consisting of hydrogen fluoride, hydrofluoric acid, and a hydrogen fluoride-containing compound, the combination may be any combination other than the combination of hydrogen fluoride and hydrofluoric acid: it may be a combination of hydrogen fluoride and a hydrogen fluoride-containing compound or a combination of hydrofluoric acid and a hydrogen fluoride-containing compound. In such a case, the reagent may be used in a solvent-free form or as a solution. In either case, the total amount, of the hydrogen fluoride and hydrogen fluoride in the hydrogen fluoride-containing compound or of hydrogen fluoride in the hydrofluoric acid and the hydrogen fluoride-containing compound, is preferably from 10wt% to 100wt%, more preferably from 10 to 80wt%, and still more preferably from 10 to 70wt% with respect to the reagent.

Given that the concentration of hydrogen fluoride falls within the above-specified range, the dephosphorylation reaction proceeds enough. If the concentration is less than 10wt%, then the dephosphorylation reaction may not proceed to completion.

While the amount of the reagent used with respect to the amount of peptide is not limited to any particular range, the reagent may be used so that the total amount of the hydrogen fluoride, hydrogen fluoride present in the hydrofluoric acid, and hydrogen fluoride present in the hydrogen fluoride-containing compound is in the range of, for example, 10 to 100000wt% with respect to the peptide.

The reaction temperature according to the present invention is preferably in the range of from -10°C to 50°C, and more preferably from 0 to 25°C. With the temperature falling within the above-specified range, the dephosphorylation reaction proceeds enough. The rate of the dephosphorylation reaction tends to become exceedingly low at temperatures lower than -10°C. The reaction tends to produce increased byproducts when carried out at temperatures higher than 50°C.

While the reaction time according to the present invention is not limited to any particular range, the reaction may be carried out over a time period of, for example, 10 minutes to 5 hours.

Peptides which are generated by eliminating phosphate groups from phosphopeptides are thus obtained in the manner described above. It should be noted that peptide bonds remain intact throughout this process.

The method of eliminating the phosphate group of a peptide in accordance with the present invention can facilitate peptide analysis.

For example, the peptides dephosphorylated in the above-described manner may be subjected, either directly or after optional fragmentation, to various analytical techniques. Alternatively, the phosphopeptides may first be optionally fragmented, and then subjected to dephosphorylation and, subsequently, to the analytical technique. The analytical technique used for this purpose is preferably mass spectrometry. One example of the peptide analysis technique, in which a peptide is fragmented prior to mass spectrometry, may be carried out in the following manner:

First, a phosphopeptide is fragmented by, for example, enzymatic digestion and the resulting mixture of the phosphopeptide fragments is subjected to mass spectrometry. Subsequently, dephosphorylation is performed and the resulting mixture of the dephosphorylated-peptide fragments is subjected to mass spectrometry. The two mass spectra are compared with each other for shift in the peaks indicative of modified peptide fragments. Also, the phosphate group can be determined from the mass difference of the shift. For example, if the peptide which contains the amino acid residues that are monophosphorylated is treated by the method of the present invention, peptide peaks having the molecular weight decreased by 80 for each phosphate group will be detected.

Subsequently, the modified peptide fragments are subjected to tandem mass spectrometry to determine modified amino acid residues and their locations.

Further, as the mass spectrometry, matrix-assisted laser desorption ionization (MALDI) and time of flight mass spectrometry (TOFMS) are preferably used.

As set forth, the method of the present invention facilitates identification of certain peptides that were considered difficult to analyze by conventional enzymatic approaches due to incomplete dephosphorylation. The method of the invention also helps reveal the functions of such peptides. Accordingly, the method of the present invention will find wider applications in the proteome analysis of phosphorylated proteins. If a protein is proven, by the use of the method of the present invention, to be involved in the onset of a particular disease, a designer compound that is targeted to the protein and specifically act on the peptide to effectively control its function may be designed on a computer as a candidate for a potential pharmaceutical product. Also, information regarding the gene encoding this protein is obtained and used to create, as a candidate for a potential pharmaceutical product, a substance to specifically control expression of the gene. The method of the present invention can facilitate creation of such candidate compounds.

### EXAMPLES

The present invention will now be described in further detail with reference to examples, which are only illustrative and do not limit the scope of the invention in any way. Unless otherwise specified, the symbol '%' is used hereinbelow to mean percentage by weight.

Phosphorylation principally takes place on three amino acid residues: serine, threonine, and tyrosine. Thus, the following three phosphopeptides were prepared and were subjected to dephosphorylation with hydrogen fluoride (HF):
1. WAGGDApSGE (SEQ ID NO: 1 of the sequence listing) (phosphorylated on a serine residue);
2. TRDIpYETDYYRK (SEQ ID NO: 3 of the sequence listing) (phosphorylated on a tyrosine residue); and
3. GFEpTVPETG-NH₂ (SEQ ID NO: 5 of the sequence listing) (phosphorylated on a threonine residue).

### [Example 1]

A phosphopeptide having the amino acid sequence of WAGGDApSGE (SEQ ID NO: 1) (Delta sleep-inducing peptide (DSIP) of *Oryctolagus cuniculus* (house rabbit), American Peptide Company Inc.) was used. This phosphopeptide is phosphorylated on a serine residue, and the phosphorylated serine residue is indicated by 'pS.'

To 100µg of the freeze-dried phosphopeptide of SEQ ID NO: 1, 50µl of 50% hydrofluoric acid (aqueous solution) was added under ice-cold condition. The reaction was allowed to proceed at 0°C for 3 hours. In a draft chamber, a stream of N₂ gas was blown to the reaction mixture to evaporate the solvent and dry the reaction product. The resulting residue was dissolved in 100µl water and was then subjected to MALDI-TOF MS (AXIMA-CFR, Shimadzu Corporation).

Fig. 1 shows a comparison of the resulting mass spectrum with other mass spectra, which confirms that dephosphorylation took place under the above-described conditions (50% HF, 0°C, 3hrs) to give a peptide having the amino acid sequence of WAGGDASGE (SEQ ID NO: 2 of the sequence listing). The top spectrum is of the reaction product obtained in the above-described reaction (signal intensity = 240mV); the middle spectrum is of a separately prepared peptide having an identical sequence to SEQ ID NO: 2 (signal intensity = 1498mV); and the bottom spectrum is of the phosphopeptide WAGGDApSGE (SEQ ID NO: 1 of the sequence listing) (signal intensity = 469mV). In each spectrum shown in Fig. 1, horizontal axis represents the mass/charge (m/z) and vertical axis represents the relative ion intensity (Int.).

In the top spectrum a peak corresponding to the molecular ions of (m/z) = 849.39 (M⁺) was detected. The molecular weight for this molecular ion peak is smaller than the molecular weight for the molecular ion peak of (m/z) = 929.34 (M⁺) detected in the bottom spectrum by an amount of 80. The difference corresponds to the difference in molecular weight between the phosphopeptide and the peptide obtained by dephosphorylating the phosphopeptide. Also, the top spectrum was highly consistent with the middle spectrum. These observations collectively indicate that the desired dephosphorylation took place without accompanying side reactions.

### [Example 2]

A phosphopeptide having the amino acid sequence of TRDIpYETDYYRK (SEQ ID NO: 3) (Insulin receptor 1142-1153 of *Homo sapiens* (human), Sigma.) was used. This phosphopeptide is phosphorylated on a tyrosine residue at the fifth position from the N-terminus, and the phosphorylated tyrosine residue is indicated by 'pY.'

To 100µg of the freeze-dried phosphopeptide of SEQ ID NO: 2, 50µl of 50% hydrofluoric acid (aqueous solution) was added under ice-cold condition. The reaction was allowed to proceed at 0°C for 3 hours. In a draft chamber, a stream of N₂ gas was blown to the reaction mixture to evaporate the solvent and dry the reaction product. The resulting residue was dissolved in 100µl water and was then subjected to MALDI-TOF MS (AXIMA-CFR, Shimadzu Corporation).

Fig. 2 shows a comparison of the resulting mass spectrum with other mass spectra, which confirms that dephosphorylation took place under the above-described conditions (50% HF, 0°C, 3hrs) to give a peptide having the amino acid sequence of TRDIYETDYYRK (SEQ ID NO: 4 of the sequence listing). The top spectrum is of the reaction product obtained in the above-described reaction (signal intensity = 253mV); the middle spectrum is of a separately prepared peptide having an identical sequence to SEQ ID NO: 4 (signal intensity = 123mV); and the bottom spectrum is of the phosphopeptide TRDIpYETDYYRK (SEQ ID NO: 3 of the sequence listing) (signal intensity = 123mV). In each spectrum shown in Fig. 2, horizontal axis represents the mass/charge (m/z) and vertical axis represents the relative ion intensity (Int.).

In the top spectrum a peak corresponding to the molecular ions of (m/z) = 1622.76 (M⁺) was detected. The molecular weight for this molecular ion peak is smaller than the molecular weight for the molecular ion peak of (m/z) = 1072.79 (M⁺) detected in the bottom spectrum by an amount of 80. The difference corresponds to the difference in molecular weight between the phosphopeptide and the peptide obtained by dephosphorylating the phosphopeptide. Also, the top spectrum was highly consistent with the middle spectrum. These observations collectively indicate that the desired dephosphorylation took place without accompanying side reactions.

### [Example 3]

A phosphopeptide having the amino acid sequence of GFEpTVPETG-NH₂ (SEQ ID NO: 5) was synthesized. This phosphopeptide is phosphorylated on a threonine residue at the fourth position from the N-terminus, and the phosphorylated threonine residue is indicated by 'pT.' Further, this phosphopeptide is amidated on a glycine residue at the C-terminus, and the amidated glycine residue is indcated by 'G-NH₂.'

To 100µg of the freeze-dried phosphopeptide of SEQ ID NO: 3, 50µl of 50% hydrofluoric acid (aqueous solution) was added under ice-cold condition. The reaction was allowed to proceed at room temperature for 3 hours. In a draft chamber, a stream of N₂ gas was blown to the reaction mixture to evaporate the solvent and dry the reaction product. The resulting residue was dissolved in 100µl water and was then subjected to MALDI-TOF MS (AXIMA-CFR, Shimadzu Corporation).

Fig. 3 shows a comparison of the resulting mass spectrum with other mass spectra, which confirms that dephosphorylation took place under the above-described conditions (50% HF, RT, 3hrs) to give a peptide having the amino acid sequence of GFETVPETG-NH₂ (SEQ ID NO: 6 of the sequence listing). In the peptide of SEQ ID NO: 6, the amidated glycine at the C-terminus indicated by 'G-NH₂.' The top spectrum is of the reaction product obtained in the above-described reaction (signal intensity = 40mV); the middle spectrum is of a separately prepared peptide having an identical sequence to SEQ ID NO: 6 (signal intensity = 325mV); and the bottom spectrum is of the phosphopeptide GFEpTVPETG-NH₂ (SEQ ID NO: 5 of the sequence listing) (signal intensity = 177mV). In each spectrum shown in Fig. 3, horizontal axis represents the mass/charge (m/z) and vertical axis represents the relative ion intensity (Int.).

In the top spectrum peaks corresponding to the molecular ions of (m/z) = 973.86 (M⁺+K), 957.90 (M⁺+Na), 935.52 (M⁺+H) were detected. The molecular weight for each of these molecular ion peaks is smaller than the molecular weight for the respective molecular ion peaks of (m/z) = 1053.64 (M⁺+K), 1037.65 (M⁺+Na), 1015.66 (M⁺+H) detected in the bottom spectrum by an amount of 80. The difference corresponds to the difference in molecular weight between the phosphopeptide and the peptide obtained by dephosphorylating the phosphopeptide. Also, the top spectrum was highly consistent with the middle spectrum. These observations collectively indicate that the desired dephosphorylation took place without accompanying side reactions.

Next, the following three phosphopeptides were subjected to dephosphorylation with hydrogen fluoride-pyridine (HF-Py):
4. WAGGDApSGE (SEQ ID NO: 1 of the sequence listing) (phosphorylated on a serine residue);
5. Ac-IpYGEF-NH₂ (SEQ ID NO: 7 of the sequence listing) (phosphorylated on a tyrosine residue); and
6. GFETVPEpTG-NH₂ (SEQ ID NO: 9 of the sequence listing) (phosphorylated on a threonine residue).

### [Example 4]

An identical phosphopeptide to that used in Example 1 was used (i.e., the amino acid sequence of WAGGDApSGE (SEQ ID NO: 1)).

To 100µl of the freeze-dried phosphopeptide of SEQ ID NO: 1, 50µl of 70% hydrogen fluoride-pyridine was added under ice-cold condition to dissolve the phosphopeptide. The reaction was allowed to proceed at 0°C for 1 hour. Subsequently, the reaction mixture was concentrated under reduced pressure to be dried up, which in turn was dissolved in 20µl water. The solution was treated with ZipTip (Millipore) prior to MALDI-TOF MS (AXIMA-CFR, Shimadzu Corporation).

The top spectrum in Fig. 4 is of the dephosphorylated peptide obtained under the above-described reaction conditions (70% HF-Py, 0°C (0 deg. C), 1hr) and having the amino acid sequence of WAGGDASGE (SEQ ID NO: 2 of the sequence listing) (signal intensity = 130mV). The bottom spectrum in Fig. 4 is of the phosphopeptide WAGGDApSGE (SEQ ID NO: 1 of the sequence listing) (signal intensity = 525mV). In each spectrum shown in Fig. 4, horizontal axis represents the mass/charge (m/z) and vertical axis represents the relative ion intensity (Int.).

In the top spectrum a peak corresponding to the molecular ions of (m/z) = 849.36 (M⁺) was detected. The molecular weight for this molecular ion peak is smaller than the molecular weight for the molecular ion peak of (m/z) = 929.34 (M⁺) detected in the bottom spectrum by an amount of 80. The difference corresponds to the difference in molecular weight between the phosphopeptide and the peptide obtained by dephosphorylating the phosphopeptide. This observation indicates that the desired dephosphorylation took place without accompanying side reactions.

### [Example 5]

A phosphopeptide having the amino acid sequence of Ac-IpYGEF-NH₂ (SEQ ID NO: 7 of the sequence listing) was synthesized. This phosphopeptide is phosphorylated on a tyrosine residue at the second position from the N-terminus, and the phosphorylated tyrosine residue is indicated by 'pY.' Also, the phosphopeptide is acetylated on an isoleucine residue at the N-terminus, and the acetylated isoleucine residue is indicated by 'Ac-I.' Furthermore, the phosphopeptide is amidated on a phenylalanine residue at the C-terminus, and the amidated phenylalanine residue is indicated by 'F-NH₂.' The same procedures were followed as in Example 4, except that the peptide of SEQ ID NO: 7 is used as a phosphopeptide to be dephosphorylated. MALDI-TOF MS analysis was performed using AXIMA-CFR (Shimadzu Corporation).

The top spectrum in Fig. 5 is of the dephosphorylated peptide obtained under the reaction conditions described in Example 5 (70% HF-Py, 0°C (0 deg. C), 1hr) and having the amino acid sequence of Ac-IpYGEF-NH₂ (SEQ ID NO: 8 of the sequence listing) (signal intensity = 68mV). In the peptide of SEQ ID NO: 8, acetylaed isoleucine at the N-terminus is indicated by 'Ac-I' and amidated phenylalanine at the C-terminus is indicated by 'F-NH₂.' The bottom spectrum in Fig. 5 is of the phosphopeptide Ac-IpYGEF-NH₂ (SEQ ID NO: 7 of the sequence listing) (signal intensity = 119mV). In each spectrum shown in Fig. 5, horizontal axis represents the mass/charge (m/z) and vertical axis represents the relative ion intensity (Int.).

In the top spectrum peaks corresponding to the molecular ions of (m/z) = 707.36 (M⁺+K), 691.38 (M⁺+Na) were detected. The molecular weight for each of these molecular ion peaks is smaller than the molecular weight for the respective molecular ion peaks of (m/z) = 787.42 (M⁺+K), 771.47 (M⁺+Na) detected in the bottom spectrum by an amount of 80. The difference corresponds to the difference in molecular weight between the phosphopeptide and the peptide obtained by dephosphorylating the phosphopeptide. This observation indicates that the desired dephosphorylation took place without accompanying side reactions.

### [Example 6]

A phosphopeptide having the amino acid sequence of GFETVPEpTG-NH₂ (SEQ ID NO: 9 of the sequence listing) was synthesized. This phosphopeptide is phosphorylated on a threonine residue at the eighth position from the N-terminus, and the phosphorylated threonine residue is indicated by 'pT.' Furthermore, the phosphopeptide is amidated on a glycine residue at the C-terminus, and the amidated glycine residue is indicated by 'G-NH₂.'

The same procedures were followed as in Example 4, except that the peptide of SEQ ID NO: 9 is used as a phosphopeptide to be dephosphorylated. MALDI-TOF MS analysis was performed using AXIMA-CFR (Shimadzu Corporation).

The top spectrum in Fig. 6 is of the dephosphorylated peptide obtained under the reaction conditions described in Example 6 (70% HF-Py, 0°C (0 deg. C), 1hr) and having the amino acid sequence of GFETVPETG-NH₂ (SEQ ID NO: 10 of the sequence listing) (signal intensity = 5.6mV). In the peptide of SEQ ID NO: 10, amidated glycine at the C-terminus is indicated by 'G-NH₂.' The bottom spectrum in Fig. 6 is of the phosphopeptide GFETVPEpTG-NH₂ (SEQ ID NO: 9 of the sequence listing) (signal intensity = 208mV). In each spectrum shown in Fig. 6, horizontal axis represents the mass/charge (m/z) and vertical axis represents the relative ion intensity (Int.).

In the top spectrum peaks corresponding to the molecular ions of (m/z) = 973.50 (M⁺+K), 957.54 (M⁺+Na) were detected. The molecular weight for each of these molecular ion peaks is smaller than the molecular weight for the respective molecular ion peaks of (m/z) = 1053.64 (M⁺+K), 1037.65 (M⁺+Na) detected in the bottom spectrum by an amount of 80. The difference corresponds to the difference in molecular weight between the phosphopeptide and the peptide obtained by dephosphorylating the phosphopeptide. This observation indicates that the desired dephosphorylation took place without accompanying side reactions.

In each of the Examples in which the above phosphopeptide was used, dephosphorylattion was performed without accompanying side reactions. Without reaction specificity or structural dependence, the method of the present invention has proven to be applicable to any peptide.

### [Example 7]

In addition to the model peptides described in Examples 1 through 6 above, α-casein (derived from bovine milk) was used for dephosphorylation. Specifically, α-casein was digested with trypsin by conventional technique to obtain a mixture of digested fragments. Using IMAC technique (Ga column), phosphopeptides were concentrated. This sample was treated with 50µl of 50% hydrofluoric acid (aqueous solution), and the dephosphorylation was carried out at 0°C for 3 hours. The spectra obtained by MALDI-TOF MS revealed that within the mass/charge range of less than 2000 (m/z), the two peaks of (m/z) = 1661.2, 1952.3 corresponding to the phosphopeptides shifted respectively to (m/z) = 1580.9, 1872.0, after treatment with hydrogen fluoride. The mass number difference between before and after the treatment was 80, indicating that each fragment was phosphorylated at one site.

While dephosphorylation of five model peptides and α-casein has been described in Examples above, the present invention is not limited to such examples and is applicable to any peptide and protein. The foregoing Examples are therefore provided by way of example only and should not be construed as limiting the scope of the invention in any way. The invention is intended to cover all changes, modifications, and equivalents that may be included within the spirit and the scope of the invention as defined by the appended claims.

In the free text of sequence listing (i.e., Description of Artificial Sequence), SEQ ID NO: 2 represents dephosphorylated delta sleep-inducing peptide, SEQ ID NO: 4 represents dephosphorylated insulin receptor 1142-1153, SEQ ID NOs: 5, 7, and 9 are synthetic phosphopeptides, and SEQ ID NOs: 6, 8, and 10 are dephosphorylated forms of synthetic phosphopeptides of SEQ ID NOs: 5, 7, and 9, respectively.

### INDUSTRIAL APPLICABILITY

As set forth, the present invention provides a method for eliminating the phosphate groups of peptides that relies not on enzymes but on a chemical treatment. The present invention also provides an effective method for peptide analysis that uses the elimination method of the invention.

## Claims

1. A method of eliminating a phosphate group of a peptide, the method comprising the use of a reagent containing at least one selected from the group consisting of hydrogen fluoride, hydrofluoric acid, and a hydrogen fluoride-containing compound.

2. The method of eliminating a phosphate group of a peptide according to claim 1, wherein the hydrogen fluoride-containing compound is hydrogen fluoride-pyridine.

3. The method of eliminating a phosphate group of a peptide according to claim 1, wherein the total amount of the hydrogen fluoride, hydrogen fluoride in the hydrofluoric acid, and hydrogen fluoride in the hydrogen fluoride-containing compound contain in the reagent is 10 to 100wt% with respect to the reagent.

4. The method of eliminating a phosphate group of a peptide according to claim 1, wherein the temperature for the elimination reaction is -10 to 50°C.

5. The method of eliminating a phosphate group of a peptide according to claim 1, wherein the elimination reaction is carried out as a liquid phase reaction or a gas phase reaction.

6. A method of analyzing a peptide, the method comprising the use of the method according to claim 1 for eliminating a phosphate group of a peptide.

7. The method of analyzing a peptide according to claim 6, comprising the use of mass spectrometry.

8. The method of analyzing a peptide according to claim 7, comprising the use of matrix-assisted laser desorption ionization (MALDI) and time of flight mass spectrometry (TOFMS).

9. A novel compound comprising a peptide identified by eliminating a phosphate group of a peptide using a reagent containing at least one selected from the group consisting of hydrogen fluoride, hydrofluoric acid, and a hydrogen fluoride-containing compound.

10. A candidate compound for a pharmaceutical product developed from the novel compound obtained in claim 9.


SEQUENCE LISTING

<110> SHIMADZU CORPORATION

<120> Method of Eliminating Phosphate Group of Peptide and Method of Analyzing Peptide

<130> GP15-03-EP

<150> JP 2003-036472
<151> 2003-02-14

<160> 10

<170> PatentIn Ver. 2.1

<210> 1
<211> 9
<212> PRT
<213> Oryctolagus cuniculus

<220>
<221> MOD_RES
<222> (7)
<223> PHOSPHORYLATION

<400> 1
Trp Ala Gly Gly Asp Ala Ser Gly Glu
 1               5

<210> 2
<211> 9
<212> PRT
<213> Artificial Sequence

<220>
<223> Description of Artificial Sequence: dephosphorylated Delta Sleep Inducing Peptide

<400> 2
Trp Ala Gly Gly Asp Ala Ser Gly Glu
 1               5

<210> 3
<211> 12
<212> PRT
<213> Homo sapiens

<220>
<221> MOD_RES
<222> (5)
<223> PHOSPHORYLATION

<400> 3
Thr Arg Asp Ile Tyr Glu Thr Asp Tyr Tyr Arg Lys
 1               5                   10

<210> 4
<211> 12
<212> PRT
<213> Artificial Sequence

<220>
<223> Description of Artificial Sequence: dephosphorylated Insulin receptor 1142-1153

<400> 4
Thr Arg Asp Ile Tyr Glu Thr Asp Tyr Tyr Arg Lys
 1               5                   10

<210> 5
<211> 9
<212> PRT
<213> Artificial Sequence

<220>
<223> Description of Artificial Sequence: synthetic phosphopeptide

<220>
<221> BINDING
<222> (4)
<223> PHOSPHORYLATION

<220>
<221> BINDING
<222> (9)
<223> AMIDATION

<400> 5
Gly Phe Glu Thr Val Pro Glu Thr Gly
1               5

<210> 6
<211> 9
<212> PRT
<213> Artificial Sequence

<220>
<223> Description of Artificial Sequence: dephosphorylated synthetic phosphopeptide

<220>
<221> BINDING
<222> (9)
<223> AMIDATION

<400> 6
Gly Phe Glu Thr Val Pro Glu Thr Gly
1               5

<210> 7
<211> 5
<212> PRT
<213> Artificial Sequence

<220>
<223> Description of Artificial Sequence: synthetic phosphopeptide

<220>
<221> BINDING
<222> (1)
<223> ACETYLATION

<220>

<221> BINDING
<222> (5)
<223> AMIDATION

<220>
<221> BINDING
<222> (2)
<223> PHOSPHORYLATION

<400> 7
Ile Tyr Gly Glu Phe
1               5

<210> 8
<211> 5
<212> PRT
<213> Artificial Sequence

<220>
<221> BINDING
<222> (1)
<223> ACETYLATION

<220>
<221> BINDING
<222> (5)
<223> AMIDATION

<220>
<223> Description of Artificial Sequence: dephosphorylated synthetic phosphopeptide

<400> 8
Ile Tyr Gly Glu Phe
1               5

<210> 9
<211> 9
<212> PRT
<213> Artificial Sequence

<220>
<221> BINDING
<222> (8)
<223> PHOSPHORYLATION

<220>
<221> BINDING
<222> (9)
<223> AMIDATION

<220>
<223> Description of Artificial Sequence: synthetic phosphopeptide

<400> 9
Gly Phe Glu Thr Val Pro Glu Thr Gly
1               5

<210> 10
<211> 9
<212> PRT
<213> Artificial Sequence

<220>
<221> BINDING
<222> (9)
<223> AMIDATION

<220>
<223> Description of Artificial Sequence: dephosphorylated synthetic phosphopeptide

<400> 10
Gly Phe Glu Thr Val Pro Glu Thr Gly
1               5